# EUROPEAN PATENT APPLICATION

(11) **EP 0 528 624 A1**
(43) Date of publication of application: **24.02.1993**
(21) Application number: 92307326.6
(22) Date of filing: 11.08.1992
(51) Int. Cl.: C12P 19/26, C07H 5/04, A61K 7/06

(54) **Di and Tri saccharides, methods of making them and hair growth compositions containing them**

(30) Priority: 14.08.1991 GB 9117523
(71) Applicant: UNILEVER PLC, London EC4P 4BQ (GB); UNILEVER N.V., NL-3013 AL Rotterdam (NL)
(72) Inventor: Critchley, Peter, Bedford MK41 8DW (GB); Singh, Suddham, Ramat, Gan 52900 (IL); Crout, David Herbert George, Cannon Park, Coventry CV4 7ED (GB)
(74) Representative: Mulder, Cornelis Willem Reinier, Dr.

(57) **Abstract**

The preparation of a di or tri saccharide in which at least one glycoside residue bears an acetylamino substituent is catalysed enzymatically using an impure enzyme preparation with N-acetylhexosaminidase activity. The compounds produced, some of which are novel, may be useful to stimulate hair growth.

## Description

### Field of the Invention

The present invention relates to di- and tri- saccharides formed from an amino sugar and a second sugar, to enzymatic methods of making such compounds and to their use.

### Background of the Invention

It is known that some di and oligosaccharides display significant biological activity. More particularly, EP-A-211610 (Unilever) discloses that di- and oligosaccharides containing uronic acid residues linked to hexosamine residues display activity in promoting hair growth.

The chemical synthesis of di and oligosaccharides frequently entails multi-step reactions in which protective groups are added and later removed in order that reaction leads to the desired products.

It has been known for many years that glycosylation reactions, in which individual saccharides become linked together, can be brought about enzymatically.

Nilsson, in Carbohydrate Research 204 1990 pages 79-83 and in WO-A-91/02806 discloses the enzymically catalysed production of regioselective disaccharide compounds, using glycosidase in the form of a comparatively crude preparation from the mollusc Chamelia gallina. Nilsson refers to disaccharides of the type

HexNAc-Hex

in which Hex denotes a hexose residue and NAc denotes an N-acetyl amino substituent thereon.

Mega et al, J. Biochem 72 1197 (1972) disclose the use of isolated-β-D-glucosaminidase to bring about transglycosylation. Their yields were very low.

They also refer briefly, on page 1203, to β-D-galactosidases from various species yielding disaccharides of the formula

Hex-HexNAc

Thus when Mega et al used galactosidases rather than glucosaminidases, they arrived at products of different structure from the type referred to by Nilsson.

### Summary of the Invention

We have discovered that some enzyme catalysts, other than those disclosed by Nilsson are equally or more effective in the formation of di and oligosaccharide compounds.

In a first aspect this invention provides a method of preparation of an oligosaccharide of the formula

HexNAc - Sacc

wherein Hex denotes a hexose residue,
NAc denotes an N-acetylamino substituent thereon and
Sacc denotes a saccharide residue
which method comprises effecting enzymatic catalysis of reaction between a saccharide as acceptor and a hexosaminyl donor, using as enzymatic catalyst an impure N-acetylhexosaminidase preparation from Aspergillus spp which also displays β-galactosidase activity.

The saccharide which is the acceptor may notably be a mono or di saccharide so that the product is a di or tri saccharide. It may in particular be a glycoside or diglycoside having a hexose or hexosamine residue to which the donor hexosaminyl group becomes attached.

The glycosyl link which is formed, in the reaction, i.e. the link in the formula

HexNAc - Sacc

may be a 1,2 linkage from the 1 position of the HexNAc donor to the acceptor but is more likely to be a 1,3, 1,4, or 1,6 linkage from the 1 position of Hex NAc to a 3, 4 or 6 position of an acceptor saccharide.

The above method has been used to prepare a number of di and tri saccharides including some which are novel compounds.

In a second aspect this invention provides novel compounds of the formula given above.

In a third aspect this invention provides the use of such novel compounds or of compounds (whether novel or not) prepared by the method of the invention, in stimulating hair growth or retarding hair loss.

The invention also provides a composition, suitable for topical application, comprising a cosmetically or pharmaceutically acceptable vehicle and a compound of the invention.

### Brief Description of the Drawings

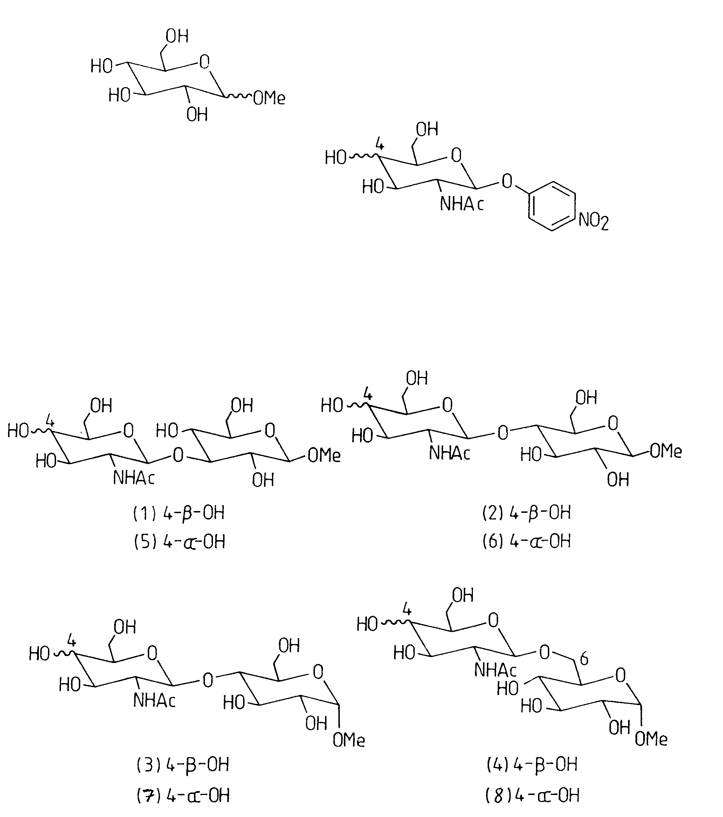

The accompanying drawing shows structural formulae for methylglucopyranoside which is an acceptor molecule, p-nitrophenylhexosamine donor molecules, and various compounds accessible by the method of this invention and referred to below.

### Detailed description

The enzymatically catalysed reaction entails transfer of a glycosyl group from the donor to the acceptor. It is therefore conveniently referred to as a "transfer reaction" and the products are conveniently referred to as "transfer products".

The enzyme preparation used in this invention has N-acetyl-hexosaminidase activity, but is not a purified enzyme of this activity. Its other activities must include β-galactosidase. The N-acetylhexosaminidase activity may be N-acetylgalactosaminidase activity. Techniques to assay for N-acetyl hexosaminidase activity have been described by Li and Li, in J. Biol Chem 245, 5153 (1970).

It is preferred to employ a β-galactosidase preparation which is sufficiently crude that it contains other glycosidase activities including N-acetylhexosaminidase. Thus the main activity displayed by the enzyme preparation may be ß-galactosidase.

Particularly preferred is a crude β-galactosidase from Aspergillus oryzae. This material contains many glycosidase activities other than that of β-galactosidase. Although these activities individually may represent less than one per cent of the β-galactosidase activity, nevertheless, the enzyme is so cheap that it represents a useful source of these minor activities. The preparation may be fractionated to enhance the specific N-acetylhexosaminidase activity. This may be carried out by ammonium sulphate fractionation, a well known technique which is disclosed, inter alia in Protein Purification, Principles and Practice by Robert K Scopes.

It was found that an 80 to 100% ammonium sulphate cut contained enhanced activity of β-N-acetylgalactosaminidase with reduced activity of β-galactosidase.

The enzymatically catalysed reaction may be brought about by incubating an aqueous solution containing the donor and acceptor substances and the enzyme preparation. At the end of the incubation period the enzyme may be destroyed by heating so that it does not catalyse decomposition.

Known techniques may be employed to isolate the products, including solvent extraction to remove non-saccharide products and high performance liquid chromatography (h.p.l.c.) to separate individual products.

The site of substitution on the acceptor has been found to depend on the enzyme used, the nature of the acceptornotably configuration at its anomeric centre, and the reaction conditions.

The donor molecule will generally be a glycoside with an aglycone leaving group.

The donor and acceptor molecules may optionally bear other substituents.

The acceptor molecule may be a glycoside or diglycoside with an aglycone group, especially an alkyl group of 1 to 4 carbon atoms, attached to an anomeric centre. In such a glycoside the saccharide residue to which the donor molecule becomes attached is preferably a hexose, optionally with acetylamino substitution. Both saccharide residues in a disaccharide may be hexoses, optionally substituted, so that the product may be represented as

HexNAc-Hex(NAc)-Hex(NAc)

where (NAc) represents an optional N-acetyl substituent.

The reaction may also be brought about using a saccharide other than a glycoside, such as glucose or sucrose.

Hexose residues in the acceptor molecules may be various hexoses, but glucose and galactose are particularly envisaged. These may be substituted, notably with acetylaminogroups.

Compounds which may be made by this invention notably include those in which HexNAc, from the donor, is
2-acetamido-2-deoxy-β-D-galactopyranosyl
or 2-acetamido-2-deoxy-β-D-glucopyranosyl.

Compounds of particular interest which may be prepared by the method of the invention include:
1. methyl 3-O-(2-acetamido-2-deoxy-β-D-galactopyranosyl)-β-D-glucopyranoside
2. methyl 4-O-(2-acetamido-2-deoxy-β-D-galactopyranosyl)-β-D-glucopyranoside
3. methyl 4-O-(2-acetamido-2-deoxy-β-D-galactopyranosyl)-α-D-glucopyranoside
4. methyl 6-O-(2-acetamido-2-deoxy-β-D-galactopyranosyl)-α-D-glucopyranoside
5. methyl 3-O-(2-acetamido-2-deoxy-β-D-glucopyranosyl)-β-D-glucopyranoside
6. methyl 4-O-(2-acetamido-2-deoxy-β-D-glucopyranosyl)-β-D-glucopyranoside
7. methyl 4-O-(2-acetamido-2-deoxy-β-D-glucopyranosyl)-α-D-glucopyranoside
8. methyl 6-O-(2-acetamido-2-deoxy-β-D-glucopyranosyl)-α-D-glucopyranoside
9. methyl 4-O-(2-acetamido-2-deoxy-β-D-galactopyranosyl)-2-acetamido-2-deoxy-β-D-glucopyranoside
10. methyl 4-O-(2-acetamido-2-deoxy-β-D-galactopyranosyl)-2-acetamido-2-deoxy-α-D-glucopyranoside
11. methyl 6-O-(2-acetamido-2-deoxy-β-D-galactopyranosyl)-2-acetamido-2-deoxy-α-D-glucopyranoside
12. β-D-GalpNAc-(1→4)-β-D-GalpNAc-(1→4)-β-D-GlupNAc OMe
13. β-D-GalpNAc-(1→3)-β-D-GalpNAc-(1→4)-β-D-GlupNAc OMe
14. 3-O-(2-acetamido-2-deoxy-β-D-galactopyranosyl)-β-D-glucopyranose
15. 3-O-(2-acetamido-2-deoxy-β-D-galactopyranosyl)-α-D-glucopyranose
16. 4-O-(2-acetamido-2-deoxy-β-D-galactopyranosyl)-β-D-glucopyranose
17. 4-O-(2-acetamido-2-deoxy-β-D-galactopyranosyl)-α-D-glucopyranose

Structural formulae for compounds 1 to 8 are illustrated in the accompanying drawing.

At least some of the above compounds (7 to 13) are novel per se.

It should be noted that two systems of terminology are in general use. An acetamido deoxypyranose is often referred to as N-acetylhexosamine.

Compounds made by the method of this invention may be incorporated into compositions for topical application to human skin. Such compositions include a pharmaceutically acceptable vehicle and many contain various other additives including perfume and preservative.

In such compositions the preferred concentration of compounds made by the method of the invention lies in the range 0.01 to 10% by weight, more preferably 0.1 to 5%. The compositions may be per se conventional for such applications being in the form for example of ointments, lotions or creams.

The selection of a cosmetically acceptable vehicle for such a composition presents a wide range of possibilities depending on the required product form of the composition. Suitable vehicles can be classified as described hereinafter.

It should be explained that vehicles are substances which can act as diluents, dispersants, or solvents for the dior tri- saccharide and which therefore ensure that it can be applied to and distributed evenly over the hair and/or scalp at an appropriate concentration. The vehicle is preferably one which can aid penetration of the di- or tri- saccharide into the skin to reach the immediate environment of the hair follicule. Compositions according to this invention can include water as a vehicle, and/or at least one cosmetically acceptable vehicle other than water.

Vehicles other than water that can be used in compositions according to the invention can include solids or liquids such as emollients, solvents, humectants, thickeners and powders. Examples of each of these types of vehicles, which can be used singly or as mixtures of one or more vehicles, are as follows:
Emollients, such as stearyl alcohol, glyceryl monoricinoleate, glyceryl monostearate, propane-1,2-diol, butane-1,3-diol, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polyethylene glycol, triethylene glycol, lanolin, castor oil, acetylatd lanolin alcohols, petroleum, mineral oil, butyl myristate, isostearic acid, palmitic acid, isopropyl linoleate, lauryl lactate, myristyl lactate, decyl oleate, myristylmyristate; propane, butane, isobutane, dimethyl ether, carbon dioxide, nitrous oxide;
Solvents, such as ethyl alcohol, methylene chloride, isopropanol, castor oil, ethylene glycol monethyl ether, diethylene glycol monobutyl ether, diethylene glycol monoethyl ether, dimethyl sulphoxide, tetrahydrofuran;
Humectants, such as glycerin, sorbitol, sodium 2-pyrrolidone -5-carboxylate, soluble collagen, dibutyl phthalate, gelatin.

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The amount of vehicle in the composition, including water if present, should preferably be sufficient to carry at least a portion of the di- or tri- saccharide to the skin. The amount of the vehicle can compromise the balance of the composition, particularly where little or no other ingredients are present in the composition. Preferably the amount of the vehicle is from 50 to 99.99%, more preferably from 90 to 99% by weight of the composition.

### Examples

In the following Examples all parts and percentages are by weight unless indicated otherwise. The enzyme used in many Examples was derived from a commercially available β-galactosidase from Aspergillus oryzae (Sigma grade XI). This enzyme preparation is known to contain various minor glycosidase activities other than the main activity which is ß-galactosidase itself.

The commercial enzyme preparation was fractionated using ammonium sulphate, as mentioned above and the 80-100% ammonium sulphate fraction was utilised.

### Example 1

p-Nitrophenyl-2-acetamido-2-deoxy-β-D-galactopyranoside, also referred to as p-nitrophenyl-N-acetyl-β-D-galactosaminide (35 mg, 0.102 mmol) and methyl β-D-glucopyranoside (199 mg, 1.02 mmol) in phosphate buffer (1.7 ml, 0.04 mol dl-3, pH 6.5)_ were heated to 45-50°C for 2 min to dissolve the substrates, cooled and incubated at 28°C, and treated with the N-acetyl-β-D-hexosaminidase from Aspergillus oryzae (Sigma, grade XI) (80-100% ammonium sulphate fraction from the crude β-galactosidase, dialysed against the above phosphate buffer). Incubation was continued for 24 hours, by which time all of the donor had been consumed. The enzyme activity was destroyed by heating the reaction mixture to 85-90°C for 10 min. The p-nitrophenol was extracted with diethyl ether. The aqueous residue was lyophilised and the disaccharide products were separated by semi-preparative h.p.l.c. using a Magnasil 5H aminopropyl column with MeCN:H₂O (81:19) as eluent and with u.v. detection at 210 nm.

Two disaccharide products only were observed in the h.p.l.c., in 49% overall yield (based on the donor glycoside p-nitrophenyl-N-acetyl-β-D-galactosaminide) and in a ratio of 4:1. These were separated and purified by h.p.l.c. and were shown, on the basis of characteristic shifts in the 13C n.m.r. spectra, to be the 1,3 (compound 1) (major isomer) and 1,4 (compound 2) (minor isomer) transfer products. The isolated yield was 34% (combined products). N.m.r. signal assignments were made using proton-proton and carbon-proton shift correlation spectroscopy (COSY). Structures were confirmed by negative ion FAB MS-MS (tandem) mass spectrometry.

### Example 2

Using the same method as in Example 1 but using methyl α-D-glucopyranoside as acceptor, a strikingly different result was obtained. In this case, only two products again were observed, in 36% overall yield. The major product (84%) was shown to be the 1,4-transfer product (compound 3) and the minor isomer (16%) was shown to be the 1,6 transfer product (compound 4). Structure assignments were made as indicated above.

### Example 3

When p-nitrophenyl-2-acetamido-2-deoxy-β-D-glucopyranoside was used as glycosyl donor, very similar results were obtained. With methyl β-D-glucopyranoside as acceptor, the 1,3 and 1,4 transfer products (compounds 5 and 6) were obtained in an overall yield of 23% and in a ratio of 55:45. With methyl α-D-glucopyranoside as acceptor, the 1,4 and 1,6 transfer products (compounds 7 and 8) were obtained in an overall yield of 17% and in ratio of 7:3.

### Example 4

Transfer reactions of p-nitrophenyl-2-acetamido-2-deoxy-β-D-galactopyranoside were carried out with methyl-2-acetamido-2-deoxy-β-D-glucopyranoside and methyl-2-acetamido-2-deoxy-α-D-glucopyranoside using β-galactosidase from Aspergillus oryzae (80-100% ammonium sulphate fraction from the crude β-galactosidase) at pH 4.5.

The h.p.l.c. results for the reaction carried out with methyl-2-acetamido-2-deoxy-β-D-glucopyranoside showed the formation of two transfer products in a 43% yield (47:53 product ratio). One transfer product was identified as compound 9, while the second product is a mixture of two trisaccharides (compounds 12 and 13).

### Example 5

Using the method of Example 4 but using methyl-2-acetamido-2-deoxy-α-D-glucopyranoside also gave two products detected by h.p.l.c., in the high yield of 91% and ratio 90:10. The transfer products were identified as compounds 10 and 11, produced in that ratio 90:10.

### Example 6

Using the method of Example 1, but using β-D-glucose as acceptor, three transfer products were obtained in a total yield of 53%. The two main products were shown to be 1,3 and 1,4 transfer products.

The same transfer products were obtained using the commercial β-galactosidase preparation in place of the 80-100% ammonium sulphate cut.

These transfer products were also obtained using β-N-acetylglucosaminidase from Aspergillus niger.

## Claims

1. A method of preparation of an oligosaccharide of the formula
HexNAc-Sacc
wherein Hex deonotes a hexose residue,
NAc denotes an N-acetylamino substitutent thereon and
Sacc denotes a saccharide residue,
which method comprises enzymatically reacting a hexosaminyl donor with a saccharide as acceptor,
using as catalyst an impure N-acetylhexosaminidase preparation from Aspergillus spp which also displays β-galactosidase activity.

2. A method according to claim 1 wherein the acceptor is a mono or di-saccharide.

3. A method according to claim 1 wherein the acceptor is a glycoside or diglycoside having a hexose or hexosamine residue to which the donor hexosaminyl group becomes attached.

4. A method according to claim 1 wherein the donor is a 2-acetamido-2-deoxy-β-D-galactopyranoside.

5. A method according to claim 1 wherein the enzyme preparation displays N-acetyl galactosaminidase activity.

6. A method according to claim 1 wherein the main activity of the enzyme preparation is β-galactosidase.

7. A composition suitable for topical application comprising a compound prepared by the method of claim 1, together with a cosmetically acceptable vehicle.

8. A compound selected from the group consisting of:
methyl 4-O-(2-acetamido-2-deoxy-β-D-glucopyranosyl)-α-D-glucopyranoside;
methyl 6-O-(2-acetamido-2-deoxy-β-D-glucopyranosyl)-α-D-glucopyranoside;
methyl 4-O-(2-acetamido-2-deoxy-β-D-galactopyranosyl)-2-acetamido-2-deoxy-β-D-glucopyranoside;
methyl 4-O-(2-acetamido-2-deoxy-β-D-galactopyranosyl)-2-acetamido-2-deoxy-α-D-glucopyranoside;
methyl 6-O-(2-acetamido-2-deoxy-β-D-galactopyranosyl)-2-acetamido-2-deoxy-α-D-glucopyranoside;
β-D-GalpNAc-(1 → 4)-β-D-GalpNAc-(1 → 4)-β-D-GlupNAc OMe;
β-D-GalpNAc-(1 → 3)-β-D-GalpNAc-(1 → 4)-β-D-GlupNAc OMe;
3-O-(2-acetamido-2-deoxy-β-D-galactopyranosyl)-β-D-glucopyranose;
3-O-(2-acetamido-2-deoxy-β-D-galactopyranosyl)-α-D-glucopyranose;
4-O-(2-acetamido-2-deoxy-β-D-galactopyranosyl)-β-D-glucopyranose; and
4-O-(2-acetamido-2-deoxy-β-D-galactopyranosyl)-α-D-glucopyranose.

9. A composition suitable for topical application comprising a compound according to claim 8 together with a cosmetically acceptable vehicle.

10. Cosmetic hair-stimulation treatment of a human which comprises applying a composition according to claim 7.

11. Cosmetic hair-stimulation treatment of a human which comprises applying a composition according to claim 9.
